(19) Europäisches Patentamt
European Patent Office
Office européen des brevets

(11) Publication number: **0 092 391 A2**

(12) **EUROPEAN PATENT APPLICATION**

(21) Application number: **83302123.1**

(22) Date of filing: **14.04.83**

(51) Int. Cl.[3]: **C 07 D 401/06**, C 07 D 401/04, A 61 K 31/445 // C07D211/58, C07D211/26

(30) Priority: **15.04.82 JP 62678/82**

(43) Date of publication of application: **26.10.83 Bulletin 83/43**

(84) Designated Contracting States: **DE FR GB IT**

(71) Applicant: **KYOWA HAKKO KOGYO CO., LTD, Ohtemachi Bldg. Ohtemachi 1-chome Chiyoda-ku, Tokyo (JP)**

(72) Inventor: **Teranishi, Masayuki, 2-13-24, Narusedai, Machida-shi Tokyo (JP)**
Inventor: **Obase, Hiroyuki, 1-12-8, Meguro, Meguro-ku Tokyo (JP)**
Inventor: **Nomoto, Yuji, 3-6-6, Naka-machi, Nagaizumi-cho Sunto-gun Shizouka-ken (JP)**
Inventor: **Shuto, Katsuichi, 410-1, Nameri, Nagaizumi-cho Sunto-gun Shitouka-ken (JP)**
Inventor: **Karasawa, Akira, 411-3, Shimonagakubo, Nagaizumi-cho Sunto-gun Shizuoka-ken (JP)**
Inventor: **Kasuya, Yutaka, 1-61, Konukainishi-machi Saiwai-ku, Kawasaki-shi Kanagawa-ken (JP)**

(74) Representative: **Lambert, Hugh Richmond et al, D. YOUNG & CO. 10 Staple Inn, London, WC1V 7RD (GB)**

(54) **Novel piperidine derivatives and pharmaceutical compositions containing same.**

(57) Novel piperidine derivatives are disclosed having hypotensive activity, as well as pharmaceutical preparations containing them. The novel compounds are of the formula

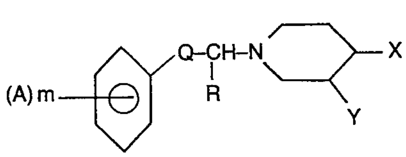

(I)

wherein A is a hydroxyl group, a halogen atom, a $C_1$–$C_5$ alkyl group, a $C_1$–$C_5$ alkoxy group, a $C_2$–$C_5$ alkenyloxy group, a $C_1$–$C_5$ alkinyloxy group, a $C_1$–$C_5$ alkylthio group, a carboxyl group, a $C_1$–$C_5$ alkoxycarbonyl group, a nitro group, an amino group, a $C_1$–$C_5$ alkylamino group, a $C_1$–$C_5$ alkanoylamino group, a sulfamoyl group, a mono- or di- ($C_1$–$C_5$ alkyl) aminosulfonyl group, a $C_1$–$C_5$ alkylsulfonyl group, a carbamoyl group, a cyano group or a trifluoromethyl group; when m is 2 or more, A may be the same or different groups, or two A groups may combine to form a $C_1$–$C_5$ alkylenedioxy group; m is O or an integer of 1–5; Q is a carbonyl group or a hydroxymethylene group; R is a hydrogen atom or an alkyl group having 1–4 carbon atoms; X and Y are defined as follows: i) when Y = H, X represents

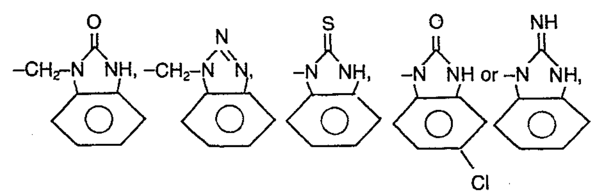

ii) when Y = $CH_3$, X represents 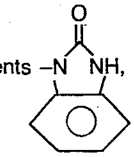

and when Y = 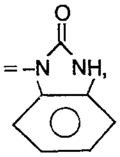

X represents a hydrogen atom and their pharmacologically acceptable acid addition salts.

EP 0 092 391 A2

# NOVEL PIPERIDINE DERIVATIVES AND PHARMACEUTICAL COMPOSITIONS CONTAINING SAME

The present invention relates to piperidine derivatives, acid addition salts thereof having hypotensive activity and pharmaceutical compositions containing the same.

Heretofore, many piperidine derivatives having a hypotensive activity have been disclosed in patent publications. For example, European Patent Publication No. 18147 published on November 12, 1980 disclosed the compounds represented by the following formula:

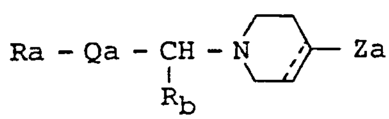

[wherein Ra is phenyl, substituted phenyl, etc.; Qa is carbonyl, methylene, hydroxymethylene, etc.; $R_b$ is hydrogen or alkyl; and Za is

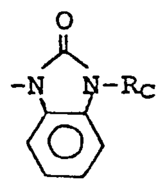

(wherein $R_c$ is hydrogen, etc.)

or

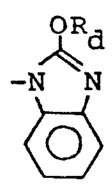

(wherein $R_d$ is alkyl) ].

Further, European Patent Publication No. 29707 published on June 3, 1981 discloses the compounds represented by the following formula:

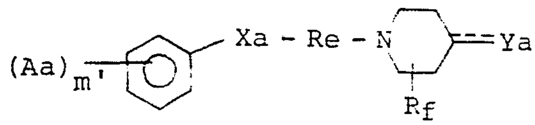

[wherein Aa is hydroxyl, halogen, lower alkyl, lower alkoxy, etc. or two Aa groups may combine to form lower alkylenedioxy; m' is 0 or an integer of 1 - 5; Xa is oxygen, sulfur, carbonyl, hydroxymethylene or methylene; Re is straight-chain alkylene having 1 - 4 carbon atoms with or without lower alkyl substituent(s); $R_f$ is hydrogen or lower alkyl; and Ya is

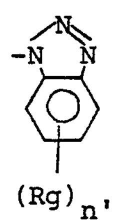 , 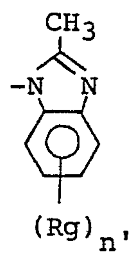 , 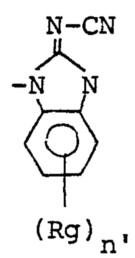 , 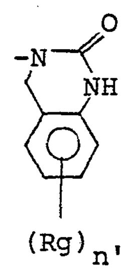 , 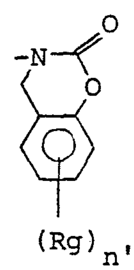 ,

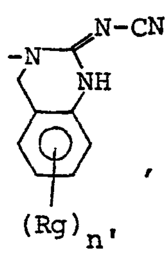 , 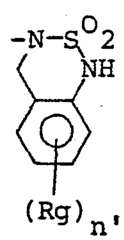 , 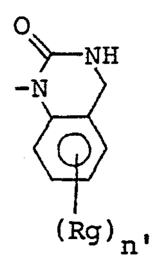 , 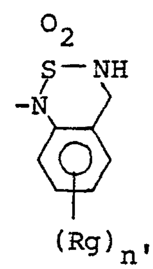 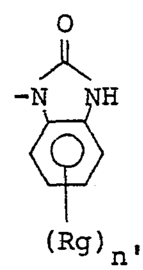

or 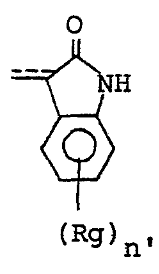

(wherein Rg is hydroxy, lower alkoxy, halogen, etc.) ].

In contrast, the present invention provides other and different piperidine derivatives having hypertensive activity, these being compounds represented by the formula:

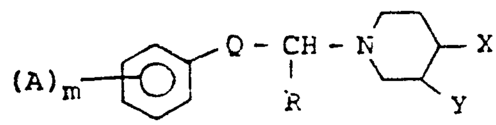

[ I ]

wherein A is a hydroxyl group, a halogen atom, a lower alkyl group, a lower alkoxy group, a lower alkenyloxy group, a lower alkinyloxy group, a lower alkylthio group, a carboxyl group, a lower alkoxycarbonyl group, a nitro group, an amino group, a lower alkylamino group, a lower alkanoylamino group, a sulfamoyl group, a mono- or di- (lower alkyl) aminosulfonyl group, a lower alkylsulfonyl group, a carbamoyl group, a cyano group or a trifluoromethyl group; when m is 2 or more, A may be the same or different groups, or two A groups may combine to form a lower alkylenedioxy group; m is 0 or an integer of 1-5; Q is a carbonyl group or a hydroxymethylene group; R is a hydrogen atom or an alkyl group having 1-4 carbon atoms; X and Y are defined as follows:

i) when Y = H, X represents

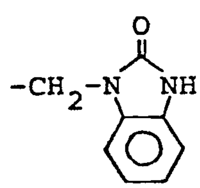 , 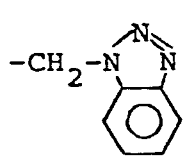 , 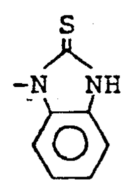 , 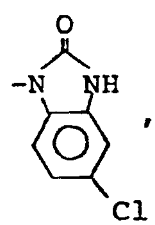 , or 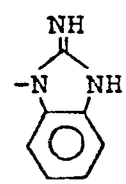

ii) when $Y = CH_3$, X represents 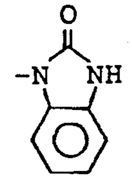 , and

iii) when Y = 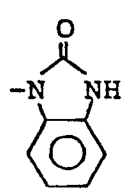 X represents a hydrogen atom

These compounds are hereinafter referred to as Compounds I. Compounds of other formulae are similarly designated.

Also covered herein are acid addition salts of the Compounds I, and pharmaceutical compositions comprising the Compounds I, or their pharmacologically acceptable acid addition salts, as an active ingredient in admixture with a pharmaceutically acceptable carrier.

The halogen atom as defined for the substituent A includes chlorine, bromine, etc. The term "lower" in the definition of the various groups for Compounds [I] means having 1 - 5 carbon atoms, particularly 1 - 3 carbon atoms.

The Compounds [I] includes all the optical isomers.

Examples of acid addition salts of Compound [I] are various inorganic acid addition salts such as hydrochloride, hydrobromide, hydroiodide, nitrate, sulfate and phosphate, and various organic acid addition salts such as formate, acetate, benzoate, maleate, fumarate, succinate, tartrate, citrate, oxalate, glyoxylate, aspartate, methanesulfonate, ethanesulfonate, propanesulfonate, methanedisulfonate, α,β-ethanedisulfonate and benzenesulfonate.

Particularly preferable Compounds [I] can be represented by the following formula [I']:

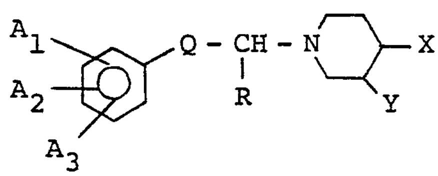

[I']

(wherein $A_1$, $A_2$ and $A_3$ are the same or different groups, and represent hydrogen atoms or lower alkoxy groups and Q, R, X and Y have the same meanings as defined above).

Typical examples of Compounds [I] are shown in Table 1, and physical properties of these compounds are shown in Tables 2 and 3.

Table 1

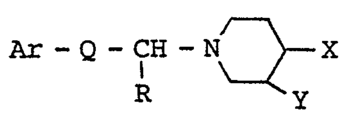

| Compound No. | Ar | Q | R | X | Y | Form | Configuration |
|---|---|---|---|---|---|---|---|
| 1 | Ar-1 | CO | $CH_3$ | X-1 | H | Fumarate | — |
| 2 | " | CHOH | " | " | " | Free | |
| 3 | Ar-2 | CO | " | " | " | " | — |

| Compound No. | Ar | Q | R | X | Y | Form | Configuration |
|---|---|---|---|---|---|---|---|
| 4 | Ar-2 | CHOH | $CH_3$ | X-1 | H | Free | |
| 5 | Ar-3 | CO | " | " | " | " | — |
| 6 | " | CHOH | " | " | " | " | threo |
| 7 | Ar-4 | CO | " | " | " | Fumarate | — |
| 8 | " | CHOH | " | " | " | Free | threo |
| 9 | " | " | " | " | " | " | erythro |
| 10 | Ar-2 | CO | " | X-2 | " | " | — |
| 11 | " | CHOH | " | " | " | " | threo |
| 12 | Ar-1 | CO | " | " | " | " | — |
| 13 | " | CHOH | " | " | " | " | threo |
| 14 | " | CO | " | X-3 | " | " | — |
| 15 | " | CHOH | " | " | " | " | threo |
| 16 | Ar-2 | CO | " | " | " | " | — |
| 17 | " | CHOH | " | " | " | " | threo |
| 18 | Ar-1 | CO | " | H | Y-1 | " | — |
| 19 | " | CHOH | " | " | " | " | threo |
| 20 | " | " | " | " | " | " | erythro |
| 21 | Ar-2 | CO | " | " | " | " | — |
| 22 | " | CHOH | " | " | " | " | threo |
| 23 | Ar-1 | CO | " | X-4 | $CH_3$ | " | trans |
| 24 | " | CHOH | " | " | " | " | trans threo |
| 25 | " | CO | " | " | " | " | cis |
| 26 | " | CHOH | " | " | " | " | cis threo |
| 27 | Ar-4 | CO | " | X-5 | H | " | — |
| 28 | " | CHOH | " | " | " | " | erythro |
| 29 | " | " | " | " | " | " | threo |

| Compound No. | Ar | Q | R | X | Y | Form | Configuration |
|---|---|---|---|---|---|---|---|
| 30 | Ar-2 | CO | $CH_3$ | X-5 | H | Free | — |
| 31 | " | CHOH | " | " | " | " | threo |
| 32 | " | " | " | " | " | " | erythro |
| 33 | Ar-1 | CO | " | " | " | " | — |
| 34 | " | CHOH | " | " | " | " | threo |
| 35 | " | CO | " | X-6 | " | " | — |
| 36 | " | CHOH | " | " | " | " | threo |

Note: Ar-1 : 3,4,5-trimethoxyphenyl
Ar-2 : 3,4-dimethoxyphenyl
Ar-3 : 2,4,6-trimethoxyphenyl
Ar-4 : 2,4,5-trimethoxyphenyl

X-1 : 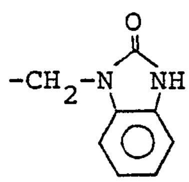

X-2 : 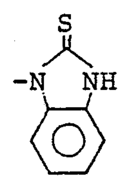

X-3 : 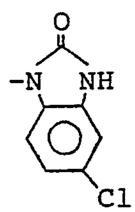

X-4 : 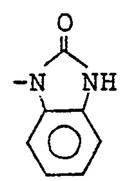

X-5 : 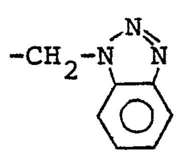

X-6 : 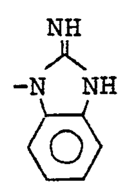

Y-1 : 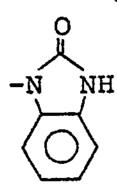

Table 2

| Compound No. | Melting point (°C) | Infrared absorption spectrum KBr ($cm^{-1}$) | Nuclear magnetic resonance spectrum | |
|---|---|---|---|---|
| | | | Solvent | δ (ppm) |
| 1 | 203 - 205 | 1699, 1678 | - | ——— |
| 2 | 202 - 204 | 1695 | $CDCl_3$ | 0.77, 1.2-3.1, 3.68, 3.83, 3.86, 4.16, 6.57, 7.2 |
| 3 | Oily | 1700, 1690 (in $CHCl_3$) | - | ——— |
| 4 | 117 - 120 | 1690, 1683$^{Sh}$ | $CDCl_3$ | 0.73, 1.0-3.4, 3.77, 3.86, 3.88, 4.18, 6.8-7.4 |
| 5 | 130 - 132 | 1688, 1680$^{Sh}$ | $CDCl_3$ | 1.2, 0.8-3.4, 3.7, 3.75, 3.8, 4.1-4.4, 6.08, 6.8-7.2, 10.1 |
| 6 | 197 - 198 | 1700 | $CDCl_3$ | 0.68, 1.1-4.0, 3.77, 3.79, 3.81, 4.97, 6.11, 7.0-7.14 |
| 7 | 193 - 196 | 1680$^{Sh}$, 1674 | - | ——— |
| 8 | 223-224.5 | 1690, 1680$^{Sh}$ | $CDCl_3$ | 0.73, 1.2-3.2, 3.77, 3.85, 3.87, 4.78, 6.50, 7.0-7.1 |
| 9 | 195 - 197 | 1690 | $CDCl_3$ | 0.84, 1.1-3.4, 3.76, 3.80, 3.84, 3.87, 5.13, 6.47, 7.0-7.1 |
| 10 | 195 - 197 | 1668 | $CDCl_3$ | 1.3, 1.5-3.4, 3.95, 6.8-8.0, 11.0 |
| 11 | 255 - 256 | 1435, 1253 | $CDCl_3$ | 0.8, 1.66-3.4, 3.86, 4.28, 6.8-7.7 |
| 12 | 197 - 198 | 1673 | $CDCl_3$ | 1.35, 1.6-3.4, 3.95, 6.8-7.5, 11.4 |
| 13 | 234 - 236 | 1455, 1115 | - | ——— |
| 14 | 166 - 168 | 1700, 1485 | $CDCl_3$ | 10.21, 7.43-6.93, 4.30, 3.95, 3.22-1.39, 1.36 |
| 15 | 230-230.5 | 1705, 1480 | $CDCl_3$ | 7.25-6.56, 4.23, 3.86, 3.83, 3.22-1.66, 0.96 |

| Compound No. | Melting point (°C) | Infrared absorption spectrum KBr ($cm^{-1}$) | Nuclear magnetic resonance spectrum | |
|---|---|---|---|---|
| | | | Solvent | δ (ppm) |
| 16 | 220 - 221 | 1700 | $CDCl_3$ | 10.46, 7.88-6.89, 4.23, 3.97, 3.24-1.79 |
| 17 | 234 - 236 | 1700, 1480 | $CDCl_3$ | 7.25-6.83, 4.25, 3.86, 3.84, 3.20-1.70, 0.73 |
| 18 | Oily | 1690 | $CDCl_3$ | 10.40, 7.50-7.05, 4.20, 3.96, 3.65-1.50, 1.32 |
| 19 | Oily | 1700 | $CDCl_3$ | 10.31, 7.27-6.57, 4.41, 4.19, 3.85, 3.03-1.96, 0.84 |
| 20 | Oily | 1690, 1480 | $CDCl_3$ | 10.21, 7.27-6.57, 4.82, 4.46, 3.84, 3.79, 3.55-1.60, 0.96 |
| 21 | Oily | 1690, 1480 | $CDCl_3$ | 9.58, 8.00-6.80, 4.80-3.82, 3.96, 3.58-1.50, 1.36 |
| 22 | Oily | 1700, 1480 | $CDCl_3$ | 10.04, 7.26-6.74, 4.62, 4.58, 3.88, 3.86, 3.79-1.75, 0.82 |
| 23 | Oily | 1690 | $CDCl_3$ | 9.87, 7.27-6.62, 4.30, 3.73, 3.30-1.60, 1.28, 1.25, 0.84 |
| 24 | Oily | 1690 | $CDCl_3$ | 10.35, 7.27-6.63, 4.20, 3.38, 3.16-1.89, 0.86, 0.84 |
| 25 | Oily | 1690 | $CDCl_3$ | 9.03, 7.53-7.04, 4.50-4.00, 3.94, 3.40-1.50, 1.30, 1,16, 0.95 |
| 26 | Oily | 1690 | $CDCl_3$ | 9.80, 7.55-7.03, 4.12, 3.96, 3.30-1.50, 0.78, 0.71 |
| 27 | Oily | 1660, 1600, 1510 | $CDCl_3$ | 8.01-6.48, 4.36, 4.00-3.94, 3.88, 3.87, 3.02-1.30, 1.22 |
| 28 | Oily | 1610, 1580, 1510, 1120 | $CDCl_3$ | 8.10-6.48, 5.14, 4.49, 4.03, 3.87, 3.84, 3.78, 3.39-1.27, 0.86 |

| Compound No. | Melting point (°C) | Infrared absorption spectrum KBr ($cm^{-1}$) | Nuclear magnetic resonance spectrum | |
|---|---|---|---|---|
| | | | Solvent | δ (ppm) |
| 29 | Oily | 1610, 1580, 1510, 1110 | $CDCl_3$ | 8.11-6.49, 4.77, 4.07, 3.87, 3.85, 3.78, 3.58-1.26, 0.74 |
| 30 | Oily | 1670, 1590, 1450 | $CDCl_3$ | 8.10-6.82, 4.48, 4.05, 3.94, 3.90, 3.00-1.38, 1.24 |
| 31 | Oily | 1610, 1590, 1510, 1130 | $CDCl_3$ | 8.12-6.83, 4.54, 4.17, 3.86, 3.45, 3.38-1.26, 0.73 |
| 32 | Oily | 1610, 1590, 1510, 1130 | $CDCl_3$ | 8.08-6.81, 4.82, 4.49, 3.85, 3.69, 3.12-1.26, 0.86 |
| 33 | 143-143.5 | 1660, 1580, 1120 | $CDCl_3$ | 8.10-7.20, 4.49, 3.98, 3.88, 3.10-1.40, 1.25 |
| 34 | 149 - 151 | 1590, 1130 | $CDCl_3$ | 8.13-6.57, 4.54, 4.14, 3.86, 3.82, 3.00-1.34, 0.76 |
| 35 | Oily | 1630, 1450 1110 | $CDCl_3$ | 7.50-6.80, 4.90-3.70, 3.40-1.60, 1.28 |
| 36 | 239 - 243 | 1630, 1450, 1110 | $CDCl_3$ | 7.50-6.95, 6.60, 4.25, 3.90, 3.20-1.88, 0.85 |

Note) Sh : Shoulder

Table 3

A : Calculated, F : Found

| Compound No. | Molecular formula | | Elemental analysis (%) C | H | N |
|---|---|---|---|---|---|
| 1 | $C_{25}H_{31}N_3O_5 \cdot C_4H_4O_4 \cdot 0.5H_2O$ | A<br>F | 60.20<br>60.44 | 6.27<br>6.16 | 7.26<br>7.41 |
| 2 | $C_{25}H_{33}N_3O_5$ | A<br>F | 65.91<br>65.74 | 7.30<br>7.52 | 9.23<br>9.19 |
| 3 | $C_{24}H_{29}N_3O_4$ | A<br>F | 68.06<br>68.00 | 6.90<br>7.11 | 9.92<br>9.83 |
| 4 | $C_{24}H_{31}N_3O_4 \cdot H_2O$ | A<br>F | 64.99<br>65.29 | 7.50<br>7.55 | 9.47<br>9.32 |
| 5 | $C_{25}H_{31}N_3O_5 \cdot H_2O$ | A<br>F | 63.68<br>63.88 | 7.05<br>7.14 | 8.91<br>9.03 |
| 6 | $C_{25}H_{33}N_3O_5$ | A<br>F | 65.91<br>65.75 | 7.30<br>7.54 | 9.23<br>9.10 |
| 7 | $C_{25}H_{31}N_3O_5 \cdot C_4H_4O_4$ | A<br>F | 61.15<br>61.10 | 6.19<br>6.09 | 7.37<br>7.44 |
| 8 | $C_{25}H_{33}N_3O_5$ | A<br>F | 65.91<br>65.70 | 7.30<br>7.43 | 9.23<br>9.34 |
| 9 | $C_{25}H_{33}N_3O_5 \cdot H_2O$ | A<br>F | 63.41<br>63.55 | 7.45<br>7.57 | 8.87<br>8.82 |
| 10 | $C_{23}H_{27}N_3O_3S$ | A<br>F | 64.92<br>64.83 | 6.39<br>6.45 | 9.87<br>9.63 |
| 11 | $C_{23}H_{29}N_3O_3S$ | A<br>F | 64.61<br>64.57 | 6.84<br>6.94 | 9.83<br>9.91 |
| 12 | $C_{24}H_{29}N_3O_3S$ | A<br>F | 63.28<br>63.54 | 6.42<br>6.47 | 9.22<br>8.96 |
| 13 | $C_{24}H_{31}N_3O_4S$ | A<br>F | 63.00<br>63.10 | 6.83<br>6.94 | 9.18<br>9.00 |
| 14 | $C_{24}H_{28}N_3O_5Cl$ | A<br>F | 60.81<br>60.93 | 5.97<br>5.93 | 8.86<br>8.99 |
| 15 | $C_{24}H_{30}N_3O_5Cl$ | A<br>F | 60.55<br>60.27 | 6.37<br>6.20 | 8.82<br>8.72 |
| 17 | $C_{23}H_{28}N_3O_4Cl$ | A<br>F | 61.94<br>61.80 | 6.34<br>6.38 | 9.42<br>9.39 |

| Compound No. | Molecular formula | | Elemental analysis (%) C | H | N |
|---|---|---|---|---|---|
| 33 | $C_{24}H_{30}N_4O_4$ | A<br>F | 65.73<br>65.83 | 6.90<br>6.87 | 12.78<br>12.96 |
| 34 | $C_{24}H_{32}N_4O_4$ | A<br>F | 65.43<br>65.22 | 7.32<br>7.32 | 12.72<br>12.88 |

Hypotensive activity of Compounds [I] will be described below, referring to Test Example.

Test Example

This test was carried out according to the method described in Spontaneously Hypertensive rats (SHR) Guidelines for Breeding, Care and Use (published by SHR committee) (1976) page 11.

Spontaneously hypertensive rats (SHR; 15 weeks old; blood pressure: 180 mmHg or higher) were used as test animals as 5 rats for one group. Compound was suspended in an aqueous 0.3% (W/V) CMC solution to make 3 mg/ml, and orally administered to the rats at a dosage of 1 ml/100 g body weight (30 mg/kg administration). Blood pressure change was measured by tail artery plethysmography (see the aforementioned literature). Maximum blood pressure reduction (mmHg) after the administration against the blood pressure before the administration is given in Table 4.

Table 4

| Compound No. | Blood pressure reduction (mmHg) | Compound No. | Blood pressure reduction (mmHg) |
|---|---|---|---|
| 1 | 48.3 | 16 | 50.0 |
| 2 | 56.3 | 17 | 51.6 |
| 4 | 73.3 | 18 | 40.0 |
| 10 | 23.3 | 19 | 38.3 |
| 11 | 16.7 | 22 | 36.7 |
| 12 | 38.3 | 23 | 65.0 |
| 13 | 30.0 | 24 | 66.7 |
| 14 | 30.0 | 26 | 58.4 |
| 15 | 83.3 | | |

Processes for preparing Compounds (I) differ depending upon the species of X, and thus will now be described according to each species of X.

(1) In case of X = 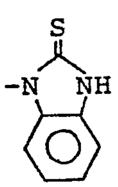 :

The Compound [I] where X = 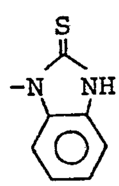 can be obtained by reacting a compound represented by the formula [II]:

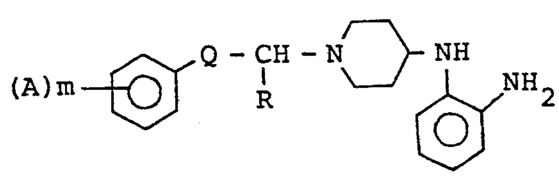

[ II ]

(wherein A, m, Q and R have the same meanings as defined above) with carbon disulfide, thiourea, thiophosgen, etc. in an inert solvent, and if necessary, further reducing the reaction product. As the inert solvent, halogenated hydrocarbons (for example, chloroform, methylene chloride, etc.) amides (for example, dimethylformamide, etc.), substituted or unsubstituted aromatic hydrocarbons (for example, benzene, toluene, etc.), lower alcohols (for example, methanol, ethanol, etc.), etc. can be used alone or in combination. The reaction is carried out in a range of 0 to 150°C, preferably between room temperature and the boiling point of the used solvent. The reaction is usually completed in 1-48 hours.

When Q is a carbonyl group, the resulting product can be further reduced to a Compound [I] whose Q is a hydroxymethylene group. The reaction can be carried out by reacting a Compound [I] whose Q is a carbonyl group with a complex metal hydride (for example, sodium borohydride, etc.) in a range of -10 to 100°C, preferably between 0°C and the boiling point of a solvent used, in a lower alcohol such as methanol, ethanol, isopropanol, etc. The reaction is usually completed in 1 - 48 hours. The reaction can also be carried out by reacting the Compound [I] with a complex metal hydride such as lithium aluminum hydride, etc. in a solvent such as tetrahydrofuran, ether, dimethoxyethane, dioxane, etc. The reaction is carried out in a range of -10°C to room temperature, and is usually completed in 1 - 12 hours.

(2) In case of X being other than 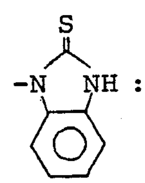 :

The Compound [I] can be obtained by reacting a piperidine derivative represented by the general formula [III]

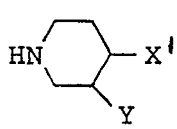

[ III ]

(wherein $X'$ represents X other than 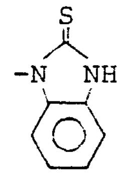 and Y has the same meaning as defined above) with a compound represented by the general formula [IV]:

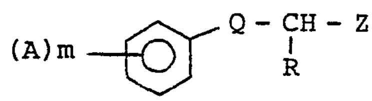 [ IV ]

(wherein A, m, Q and R have the same meanings as defined above, and Z is an eliminable group) in an inert solvent, and, if necessary, further reducing the reaction product, and, if necessary, leaving the protective group.

Z of Compound [IV] includes a halogen atom (for example, chlorine, bromine and iodine), an alkylsulfonyloxy group (for example, methanesulfonyloxy, etc.), an arylsulfonyloxy group (for example, benzenesulfonyloxy, para-toluenesulfonyloxy, etc.), etc. As the inert solvent, ketones (for example, acetone, etc.), halogenated hydrocarbons (for example, chloroform, methylene chloride, etc.), amides (for example, dimethylformamide, etc.), substituted or unsubstituted aromatic hydrocarbons (for example, benzene, toluene, chlorobenzene, etc.), lower alcohols (for example, methanol, ethanol, isopropanol, etc.), etc. can be used alone or in combination.

The reaction is carried out in a range of 0 to 150°C, preferably between room temperature and the boiling point of a solvent used, depending on the reactivity of group Z to be exchanged. The reaction is usually completed in 1 - 48 hours.

Generally, the presence of a base can make the reaction proceed more smoothly. The base to be used in the reaction includes a lower alcoholate (for example, sodium methylate, sodium ethylate, etc.), an alkali hydroxide (for example, sodium hydroxide, etc.), an alkali carbonate (for example, sodium carbonate, potassium

carbonate, etc.), a tertiary amine (for example, triethylamine, pyridine, etc.), etc.

An appropriate amount of the base to be used in the reaction is about 1.0 - 1.2 times the equivalent of Compound [III] (when an acid addition salt of Compound [III] is used, it goes without saying that additional base enough to neutralize the acid is required). Use of a reaction promoter such as potassium iodide, etc. is preferable for smooth progress of the reaction.

When Q is a carbonyl group, the resulting product can be further reduced to a Compound [I] whose Q is a hydroxymethylene group. The reaction can be carried out by reacting a Compound [I] whose Q is a carbonyl group with a complex metal hydride (for example, sodium borohydride, etc.) in a range of -10 to 100°C, preferably between 0°C and the boiling point of a solvent used, in a lower alcohol such as methanol, ethanol, isopropanol, etc. The reaction is usually completed in 1 - 48 hours. The reaction can also be carried out by reacting the said Compound [I] with a complex metal hydride such as lithium aluminum hydride, etc. in a solvent such as tetrahydrofuran, ether, dimethoxyethane, dioxane, etc. The reaction is carried out between -10°C and room temperature, and is usually completed in 1 - 12 hours.

The reaction can also be carried out by catalytic reduction of a Compound [I] whose Q is a carbonyl group in the presence of a hydrogenating catalyst such as palladium carbon and platinum oxide in a lower alcohol such as methanol and ethanol, a lower fatty acid such as acetic acid, water, dioxane, or their mixed solvent under a hydrogen atmosphere. The reaction temperature is usually between room temperature and 50°C, and the reaction time is usually about 1 - 48 hours. The reaction can be carried out in an open atmosphere, or under pressure in a closed vessel.

There are some new compounds among Compounds [III] as raw materials for synthesis of Compound [I], and processes for preparing such novel Compounds [III] are described below:

Among Compounds [III], novel ones are compounds wherein Y = H and $X^1$ = 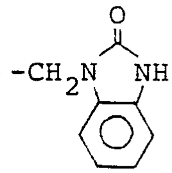 , 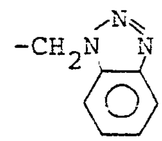 , or 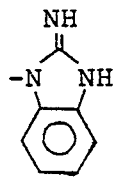 , compounds wherein $Y = CH_3$ and $X^1$ = 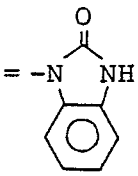 , and compounds wherein Y = 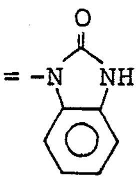 and $X^1$ = H.

The processes are described below in this order:

(a) Process for preparing Compound [III] wherein Y = H and $X^1$ = 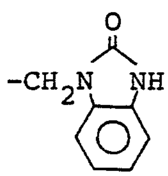 , which is hereinafter referred to as Compound [IIIa]:

The Compound [IIIa] can be prepared in the following manner.

First of all, a 4-aminomethylpiperidine derivative represented by the general formula [V]:

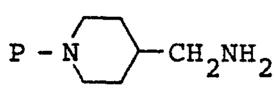

[ V ]

(wherein P is a protective group for an amino group) is reacted with 2-chloronitrobenzene in the presence of a base to prepare a compound represented by the general formula [VI]:

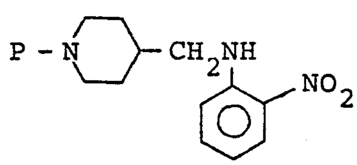

[ VI ]

(wherein P has the same meaning as defined above). Examples of said P (a protective group for an amino group) include an acyl group (for example, acetyl, benzoyl, etc.), an alkyloxycarbonyl group (for example, tert-butoxycarbonyl, ethoxycarbonyl, etc.), benzyl, tosyl, etc.

The reaction for obtaining the Compound [VI] can be carried out at a temperature of room temperature to about 200°C in an inert solvent such as dimethylformamide, dimethylsulfoxide, etc. The base to be used in the reaction includes potassium carbonate, sodium carbonate, sodium hydroxide, triethylamine, 1,5-diazabicyclo[5. 4. 0] unde-5-cene (DBU), etc. An appropriate amount of the base is about 1 to 1.5 times the equivalent of the Compound [V].

Then, the nitro group of the Compound [VI] is reduced to an amino group to give a compound represented by the general formula [VII]:

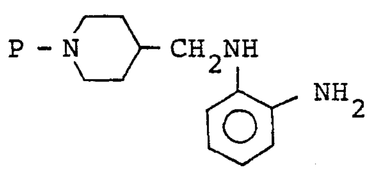

[ VII ]

(wherein P has the same meaning as defined above).

The reduction can be carried out according to the ordinary method, for example, by catalytic reduction using palladium-carbon, Raney nickel, etc. as a catalyst, or a method using tin (or a tin compound) in an acidic condition, or a method using a complex metal hydride such as lithium aluminum hydride, etc.

Then, the Compound [VII] is reacted with a carbonate derivative to give a compound represented by the general formula [VIII]

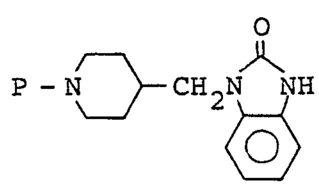

[ VIII ]

(wherein P has the same meaning as defined above). Preferable carbonate derivatives are carbonyl halide (for example, phosgene, trichloromethyl chloroformate, alkyl chlorocarbonate, etc.), 1,1'-carbonyldiimidazole, urea, etc.

The foregoing reactions are all carried out according to the ordinary method, and the reaction using 1,1'-carbonyldiimidazole is described below:

The reaction is carried out in an inert solvent such as halogenated hydrocarbon (for example, methylene chloride and chloroform), ether (for example, ethyl ether, tetrahydrofuran and dioxane), acetonitrile, dimethylformamide, dimethylsulfoxide, etc., preferably with stirring. An appropriate amount of 1,1'-carbonyldiimidazole is 1.0 - 2.0 times the equivalent of the Compound [VII]. The reaction is carried out at a temperature between room temperature and the boiling point of a solvent, and is usually completed in 1 - 3 hours at the boiling point and in 10 - 15 hours at room temperature.

Finally, the Compound [VIII] is subjected to reaction usually used to leave the protective group for an amino group and converted to the Compound [IIIa].

(b) Process for preparing a Compound [III] wherein Y = H and $X^1$ = 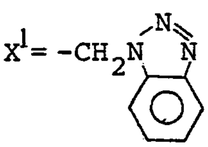 , which is hereinafter referred to as Compound [IIIb].

The Compound [IIIb] can be prepared, for example, in the following manner.

First of all, a compound represented by the general formula [VII] is diazotized to give a compound represented by the general formula [IX]:

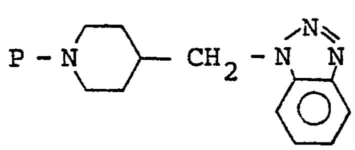

[ IX ]

(wherein P has the same meaning as defined above). As the diazotizing agent, sodium nitrite, isoamyl nitrite, etc. are used. The solvent to be used in the reaction includes water, methanol, chloroform, etc. The reaction is carried out at temperature of -10°C to the boiling point of a solvent used, preferably between about -10°C and room temperature. Depending upon the species of the diazotizing agent, it is preferable to carry out the reaction in the presence of an acid such as acetic acid, hydrochloric acid, trifluoroacetic acid, etc.

Then, the protective group for an amino group is left under the same conditions as in preparation of the Compound [IIIa] to give a Compound [IIIb].

(c) Process for preparing a Compound [III], wherein Y = H and $X^1$ = 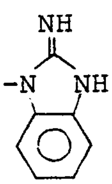 , which is hereinafter referred to as Compound [IIIc]:

The Compound [IIIc] can be prepared in the following manner. A compound represented by the general formula:

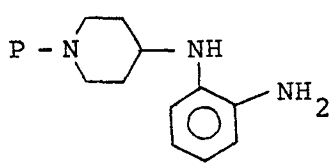

[X]

(wherein P has the same meaning as defined above) is reacted with phenacyl thiocyanate to give a compound represented by the general formula [XI]:

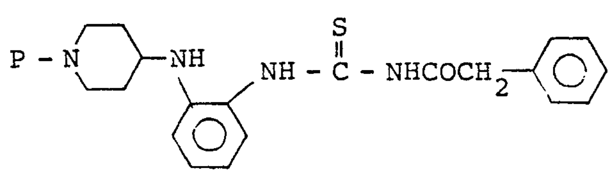

[XI]

(wherein P has the same meaning as defined above). The Compound [X] can be prepared according to the method disclosed in Japanese Published Unexamined Patent Application 13780/1976. The reaction for obtaining the Compound [XI] can be carried out at a temperature of about 0 to 60°C in an inert solvent such as dimethylformamide, dimethylsulfoxide, tetrahydrofuran, etc.

Then, the Compound [XI] is treated with a base to produce a compound represented by the general formula [XII]:

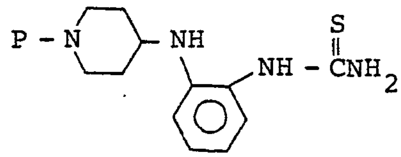

[ XII ]

(wherein P has the same meaning as defined above). The reaction is carried out at a temperature of 0 to 60°C in a solvent such as water, ethanol, dimethylformamide, etc. The base to be used in the reaction can be exemplified by aqueous ammonia, an aqueous sodium hydroxide solution, etc.

Then, the Compound [XII] is reacted with an alkylating agent to produce a compound represented by the general formula [XIII]:

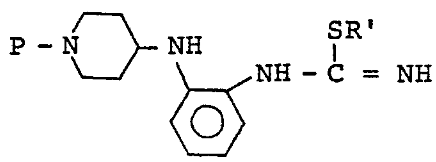

[ XIII ]

(wherein P has the same meaning as defined above and R' represents a lower alkyl group). The reaction can be carried out at a temperature of about 0 to 60°C in an inert solvent such as ethanol, dimethylformamide, chloroform, tetrahydrofuran, etc., and an appropriate alkylating agent is exemplified by dimethyl sulfate, methyl iodide, ethyl iodide, etc.

Then, the Compound [XIII] is converted to a compound represented by the general formula [XIV]:

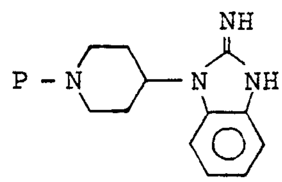

[ XIV ]

(wherein P has the same meaning as defined above) by ring closure. The reaction can be carried out in the presence of a base at a temperature of about 0°C to room temperature in a solvent such as water, methanol, tetrahydrofuran, etc. The base to be used in the reaction includes not only sodium hydroxide, potassium hydroxide, triethylamine, etc., but also more preferably anion exchange resin, etc. The reaction can also be carried out in the presence of a silver compound and a mercury compound. The silver compound can be exemplified by silver acetate, silver trifluoroacetate, silver perchlorate, etc., and the mercury compound can be exemplified by mercury acetate, etc. The solvent to be used in the reaction can be exemplified by methanol, chloroform, dimethylformamide, etc. taking no part in the reaction, and the reaction can be carried out at a temperature of 0 to 100°C.

Finally, the Compound [XIV] is subjected to a protective group-leaving reaction similar to that used in obtaining the Compound [IIIa] to produce the Compound [IIIc].

(d) Process for preparing a Compound [III] wherein $Y = CH_3$ and $X^1 =$ 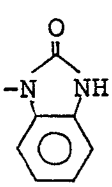 , which is hereinafter referred to as Compound [IIId]:

The Compound [IIId] can be prepared in the following manner.

First of all, a compound represented by the general formula [XV]:

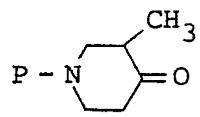

[ XV ]

(wherein P has the same meaning as defined above) [whose synthetic method is disclosed in J. Med. Pharm. Chem., 5, 913 (1962)]: is reacted with hydroxylamine in methanol to derive a compound represented by the general formula [XVI]:

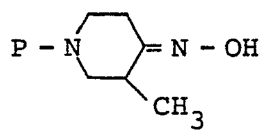

[ XVI ]

(wherein P has the same meaning as defined above), and then the oxime group of the Compound [XVI] is reduced to an amino group to give a compound represented by the general formula [XVII]:

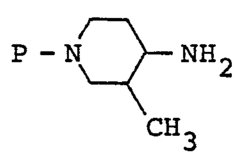

[ XVII ]

(wherein P has the same meaning as defined above). The reduction can be carried out according to the ordinary method, for example, by catalytic reduction using palladium-carbon, Raney nickel, etc. as a catalyst, or by a method using a complex metal compound (for example, aluminum hydride, etc.).

The Compound [XVII] can also be prepared by reducing the Compound [XV] with sodium borohydride, etc. in the presence of ammonium acetate in a solvent such as methanol, etc.

Further, the Compound [XVII] is subjected to a series of reactions to produce the Compound [VIII] from the

Compound [V], whereby a compound represented by the general formula [XVIII]:

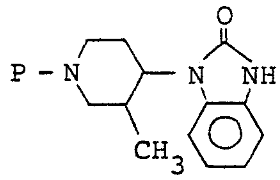

[ XVIII ]

(wherein P has the same meaning as defined above) is prepared.

Further, P of the Compound [VIII] is left in the manner as described for the Compound [IIId].

(e) Process for preparing a Compound [III] wherein

Y = 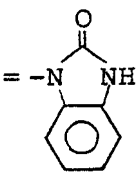 and X = H, which is hereinafter referred to as Compound [IIIe].

First of all, 4-ethoxycarbonyl-3-oxopiperidine derivative represented by the general formula [XIX]:

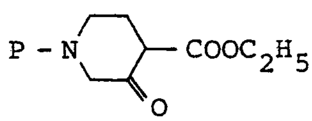

[ XIX ]

(wherein P has the same meaning as defined above) is reacted with o-phenylenediamine under a dehydrating condition to produce a compound represented by the general formula [XX]:

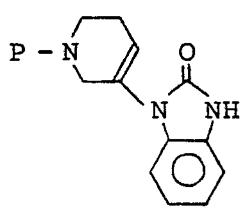

[ XX ]

(wherein P has the same meaning as defined above). The solvent to be used in the reaction is exemplified by toluene, xylene, etc. The reaction can be carried out while dehydrating under reflux using a Dean-Stark apparatus, etc.

Further, the double bond of Compound [XX] is reduced to a compound represented by the general formula [XXI]:

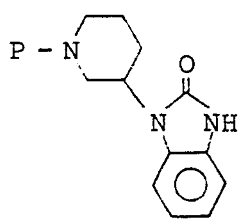

[ XXI ]

(wherein P has the same meaning as defined above). The reduction can be carried out by catalytic reduction using palladium-carbon, Raney nickel, etc. as a catalyst.

Finally, the protective group for the amino group of the Compound [XXI] is left under a condition similar to that for leaving the protective group for an amino group to obtain the Compound [IIIa], whereby the Compound [IIIe] can be prepared. By proper selection of the protective group P in the Compound [XX], the reduction of double bond and leaving of the protective group P can be carried out at the same time, whereby the Compound [IIIe] can be obtained from the Compound [XX] through one step. Such protective group P is exemplified by benzyl, etc.

Isolation and purification of Compound [I] and the said intermediates can be carried out according to the ordinary procedures in the field of organic chemistry, for example, by concentration, extraction, recrystallization, chromatography, etc.

The Compounds [I] and pharmacologically acceptable acid addition salts thereof have pharmacological activities, especially hypotensive activity and are expected to be useful as medicaments. Examples of pharmacologically acceptable acid addition salts of Compound [I] are the same as those of acid addition salts of Compound [I]. Thus, the present invention also relates to a pharmaceutical composition which comprises at least one pharmaceutically acceptable carrier, and a Compound [I] or a pharmacologically acceptable acid addition salt thereof. A pharmacologically acceptable acid addition salt of Compound [I] may be obtained by reacting Compound [I] with a suitable acid in a suitable solvent such as ethanol.

The pharmaceutical compositions of the present invention are described below.

It is obvious from the foregoing various experimental data that Compounds [I] have a hypotensive activity.

In view of the hypotensive activity, the compounds of the present invention may be used in various pharmaceutical forms for administration. Pharmaceutical compositions of the present invention are prepared by uniformly mixing an effective amount of the compound in the form of a base or an acid addition salt as an active ingredient with a pharmaceutically acceptable carrier. According to the pharmaceutical forms suitable for administration, the carrier may take various forms. It is desirable that the pharmaceutical compositions are in single administration form suitable for administration per os or by injection.

In preparation of the compositions for oral administration, any useful pharmaceutical carrier may be used. For example, water, glycols, oils, alcohols, etc. may be used to prepare oral liquid preparations such as suspensions and syrups, and excipients, lubricants, binders, disintegrators, etc. may be used to prepare powders, pills, capsules and tablets. Examples of the carriers are glucose and lactose as the excipients, starch and sodium alginate as the disintegrators, magnesium stearate, paraffin sulfate

and talc as the lubricants, and syrup, ethanol and gelatin as the binders. The active ingredient is orally administered in a dose of 1 - 100 mg, particularly 10 - 60 mg, per day for an adult.

Embodiments of the present invention are described below, referring to Examples, and the physical properties of the desired product in each Example are shown in Tables 2 and 3.

Examples of preparing the individual intermediates are shown as Reference Examples.

Example 1

A solution containing 1.7 g of 4-(1,3-dihydro-2-oxo-2H-benzimidazol-1-yl) methylpiperidine hydrochloride, 1.93 g of 2-bromo-1-(3,4,5-trimethoxyphenyl) propanone and 1.3 g of triethylamine in 50 ml of methanol was stirred at room temperature for 2 days. The solvent was removed therefrom by distillation, and the residue was distributed with ethyl acetate and water. The organic layer was washed with water and dried over sodium sulfate, and the solvent was removed therefrom by distillation. The residue was dissolved in 10 ml of isopropanol, 720 mg of fumaric acid was added, and the mixture was stirred. The crystals thus formed were collected therefrom by filtration. The crystals were recrystallized from isopropanol to obtain 2.29 g of 1-[2-oxo-2-(3,4,5-trimethoxyphenyl-1-methylethyl]-4-(1,3-dihydro-2-oxo-2H-benzimidazol-1-yl) methylpiperidine fumarate (compound 1)

Example 2

The compound obtained in Example 1 was mixed with an aqueous sodium hydrogen carbonate solution and ethyl acetate by shaking, and the organic layer was separated. The organic layer was washed with water and dried over magnesium sulfate. The solvent was removed therefrom by distillation. The residual oily matter (free compound of compound 1) was used as a starting material for Example 2.

At first, 1.6 g of free compound of compound 1 was dissolved in 30 ml of methanol, and 0.4 g of sodium borohydride was added to the solution by portions at room temperature. After the addition, the mixture was stirred at the same temperature for one hour, and the solvent was removed therefrom by distillation. Water was added to the residue, and the mixture was stirred. The deposited crystals were collected therefrom by filtration, dried and recrystallized from isopropanol to obtain 874 mg of threo-1-[2-hydroxy-2-(3,4,5-trimethoxyphenyl)-1-methylethyl]-4-(1,3-dihydro-2-oxo-2H-benzimidazol-1-yl)-methylpiperidine (compound 2).

Example 3

A solution containing 2.0 g of 4-(1,3-dihydro-2-oxo-2H-benzimidazol-1-yl)-methylpiperidine hydrochloride, 2.3 g of 2-bromo-1-(2,4,6-trimethoxyphenyl) propanone, and 1.6 g of triethylamine in 15 ml of dimethylformamide was stirred at room temperature for 2 days. Then, the solution was concentrated under reduced pressure, and the residue was distributed with ethyl acetate and water. The organic layer was washed with water and dried over magnesium sulfate, and the solvent was removed therefrom by distillation. The residue was purified by silica gel chromatography (eluent, chloroform : methanol = 20 : 1) to obtain 1.44 g of 1-[2-oxo-2-(2,4,6-trimethoxyphenyl)-1-methylethyl]-4-(1,3-dihydro-2-oxo-2H-benzimidazol-1-yl) methylpiperidine (compound 5).

Example 4

A suspension containing 300 mg of lithium aluminum hydride in 30 ml of dried tetrahydrofuran was cooled to 0°C. Then, 1.1 g of the compound obtained in Example 3 was added to the suspension by portions. After the addition, the temperature of the suspension was returned to room temperature, and it was stirred for 2 hours. The reaction mixture was then poured into ice, and extracted with chloroform. The extract was washed with water, dried over magnesium sulfate, and concentrated. The residue was recrystallized from ethyl acetate to obtain 750 mg of threo-1-[2-hydroxy-2-(2,4,6-trimethoxyphenyl)-1-methylethyl]-4-(1,3-dihydro-2-oxo-2H-benzimidazol-1-yl) methylpiperidine (compound 6).

Example 5

A solution containing 7.6 g of 1-[2-(3,4-dimethoxyphenyl)-2-oxo-1-methylethyl]-4-(2-aminoanilino) piperidine triacetate, 4.0 g of triethylamine and 35 ml of carbon disulfide in 150 ml of ethanol was refluxed for 6 hours.

The solution was concentrated, and the residue was distributed with ethyl acetate and an aqueous sodium hydrogen carbonate. The organic layer was washed with water, dried over magnesium sulfate, and concentrated. The residue was purified by silica gel chromatography (eluent, chloroform : ethyl acetate = 10 : 3) to obtain 4.3 g of 1-[2-(3,4-dimethoxyphenyl)-2-oxo-1-methylethyl)-4-(1,3-dihydro-2-thioxo-2H-benzimidazol-1-yl) piperidine (compound 10).

Example 6

At first, 2.6 g of the compound obtained in Example 5 was reacted with 0.6 g of sodium borohydride in 170 ml of methanol in the same manner as in Example 2. Then, the reaction mixture was post-treated also in the same manner to obtain 2.04 g of threo-1-[2-(3,4-dimethoxyphenyl)-2-hydroxy-1-methylethyl]-4-(1,3-dihydro-2-thioxo-2H-benzimidazol-1-yl) piperidine (compound 11) (as recrystallized from dimethylformamide-methanol).

Example 7

A solution containing 3.0 g of 3-(1,3-dihydro-2-oxo-2H-benzimidazol-1-yl) piperidine hydrochloride, 3.6 g of 2-bromo-1-(3,4,5-trimethoxyphenyl) propanone, and 3.6 ml of triethylamine in 80 ml of methanol was stirred at room temperature for 14 hours. After the same post-treatment as in Example 3, 4.4 g of 1-[2-oxo-2-(3,4,5-trimethoxyphenyl)-1-methylethyl]-3-(1,3-dihydro-2-oxo-2H-benzimidazol-1-yl) piperidine (compound 18) was obtained.

Example 8

At first, 2.8 g of the compound obtained in Example 7 was dissolved in 80 ml of ethanol, and 380 mg of sodium borohydride was added to the solution. The mixture was stirred at room temperature for 4 hours. The solvent was removed therefrom by distillation, and the residue was distributed with water and ethyl acetate. The organic layer was washed with water and dried over sodium sulfate, and the solvent was removed therefrom by distillation.

The residue was purified by silica gel chromatography (eluent, chloroform : methanol = 50 : 1), and the fractions eluted first were collected. The solvent was removed therefrom by distillation to obtain 2.1 g of threo-1-[2-hydroxy-2-(3,4,5-trimethoxyphenyl)-1-methylethyl]-3-(1,3-dihydro-2-oxo-2H-benzimidazol-1-yl) piperidine (compound 19).

Example 9

Fractions eluted second by the column chromatography of Example 8 were collected, and the solvent was removed therefrom by distillation to obtain 0.4 g of erythro-1-[2-hydroxy-2-(3,4,5-trimethoxyphenyl)-1-methylethyl]-3-(1,3-dihydro-2-oxo-2H-benzimidazol-1-yl) piperidine (compound 20).

Example 10

At first 1.0 g of 3-(1,3-dihydro-2-oxo-2H-benzimidazol-1-yl) piperidine hydrochloride, 1.1 g of 2-bromo-1-(3,4-dimethoxyphenyl) propanone, and 1.4 ml of triethylamine in 20 ml of methanol were subjected to reaction in the same manner as in Example 7, and then the reaction mixture was also subjected to the same post-treatment to obtain 1.4 g of 1-[2-oxo-2-(3,4-dimethoxyphenyl)-1-methylethyl]-3-(1,3-dihydro-2-oxo-2H-benzimidazol-1-yl) piperidine (compound 21).

Example 11

At first, 1.2 g of the compound obtained in Example 10 was reacted with 0.19 g of sodium borohydride in 40 ml of ethanol in the same manner as in Example 8, and then the reaction mixture was also subjected to the same post-treatment to obtain 0.80 g of threo-1-[2-hydroxy-2-(3,4-dimethoxyphenyl)-1-methylethyl]-3-(1,3-dihydro-2-oxo-2H-benzimidazol-1-yl) piperidine (compound 22).

Example 12

At first, 3.0 g of 4-(5-chloro-1,3-dihydro-2-oxo-2H-benzimidazol-1-yl) piperidine hydrobromide, 2.7 g of 2-

bromo-1-(3,4,5-trimethoxyphenyl) propanone, and 2.8 ml of triethylamine were stirred in 40 ml of methanol at room temperature, and then the reaction mixture was subjected to the same post-treatment as in Example 3 to obtain 3.7 g of 1-[2-oxo-2-(3,4,5-trimethoxyphenyl)-1-methylethyl]-4-(5-chloro-1,3-dihydro-2-oxo-2H-benzimidazol-1-yl) piperidine (compound 14).

Example 13

At first, 2.2 g of the compound obtained in Example 12 was reacted with 0.26 g of sodium borohydride in 90 ml of ethanol in the same manner as in Example 2, and then the reaction mixture was also subjected to the same post-treatment to obtain 1.7 g threo-1-[2-hydroxy-2-(3,4,5-trimethoxyphenyl)-1-methylethyl]-4-(5-chloro-1,3-dihydro-2-oxo-2H-benzimidazol-1-yl) piperidine (compound 15).

Example 14

A solution containing 3.0 g of 4-(5-chloro-1,3-dihydro-2-oxo-2H-benzimidazol-1-yl) piperidine hydrobromide, 2.5 g of 2-bromo-1-(3,4-dimethoxyphenyl) propanol, and 2.8 ml of triethylamine in 40 ml of methanol was stirred at room temperature for 15 hours. Crystals deposited were collected therefrom by filtration and dried to obtain 2.9 g of 1-[2-oxo-2-(3,4-dimethoxyphenyl)-1-methylethyl]-4-(5-chloro-1,3-dihydro-2-oxo-2H-benzimidazol-1-yl) piperidine (compound 16).

Example 15

At first, 1.0 g of the compound obtained in Example 14 was reacted with 0.13 g of sodium borohydride in 110 ml of ethanol in the same manner as in Example 2, and then the reaction mixture was also subjected to the same post-treatment to obtain 1.1 g of threo-1-[2-hydroxy-2-(3,4-dimethoxyphenyl)-1-methylethyl]-4-[5-chloro-1,3-dihydro-2-oxo-2H-benzimidazol-1-yl) piperidine (compound 17).

Example 16

A solution containing 2.0 g of trans-3-methyl-4-(1,3-dihydro-2-oxo-2H-benzimidazol-1-yl) piperidine hydrochloride, 2.3 g of 2-bromo-1-(3,4,5-trimethoxyphenyl) propanone and 2.8 ml of triethylamine in 30 ml of methanol was subjected to reaction in the same conditions as in Example 3, and then the reaction mixture was subjected to the same post-treatment to obtain 2.7 g of trans-1-[2-oxo-2-(3,4,5-trimethoxyphenyl)-1-methylethyl]-3-methyl-4-(1,3-dihydro-2-oxo-2H-benzimidazol-1-yl) piperidine (compound 23).

Example 17

At first, 1.5 g of the compound obtained in Example 16 was reacted with 0.19 g of sodium borohydride in 30 ml of ethanol in the same manner as in Example 8, and then the reaction mixture was also subjected to the same post-treatment to obtain 1.3 g of trans-threo-1-[2-hydroxy-2-(3,4,5-trimethoxyphenyl)-1-methylethyl]-3-methyl-4-(1,3-dihydro-2-oxo-2H-benzimidazol-1-yl) piperidine (compound 24).

Example 18

At first, 2.0 g of cis-3-methyl-4-(1,3-dihydro-2-oxo-2H-benzimidazol-1-yl) piperidine hydrochloride, 2.3 g of 2-bromo-1-(3,4,5-trimethoxyphenyl) propanone and 2.8 ml of triethylamine were subjected to reaction in 30 ml of methanol in the same manner as in Example 3, and then the reaction mixture was also subjected to the same post-treatment to obtain 1.6 g of cis-1-[2-oxo-2-(3,4,5-trimethoxyphenyl)-1-methylethyl]-3-methyl-4-(1,3-dihydro-2-oxo-2H-benzimidazol-1-yl) piperidine (compound 25).

Example 19

At first, 1.3 g of the compound obtained in Example 18 was reacted with 0.20 g of sodium borohydride in 30 ml of ethanol in the same manner as in Example 8, and then the reaction mixture was also subjected to the same

post-treatment to obtain 0.70 g of cis-threo-1-[2-hydroxy-2-(3,4,5-trimethoxyphenyl)-1-methylethyl]-3-methyl-4-(1,3-dihydro-2-oxo-2H-benzimidazol-1-yl) piperidine (compound 26).

Example 20

At first, 1.3 g of 4-(1H-benzotriazol-1-yl) methylpiperidine obtained in Reference Example 5, 2.0 g of 2-bromo-1-(3,4,5-trimethoxyphenyl) propanone, and 1.2 ml of triethylamine were subjected to reaction in 30 ml of methanol in the same manner as in Example 3, and then the reaction mixture was also subjected to the same post-treatment to obtain 0.48 g of 1-[2-oxo-2-(3,4,5-trimethoxyphenyl)-1-methylethyl-4-(1H-benzotriazol-1-yl) methylpiperidine (compound 33).

Example 21

At first, 2.6 g of 4-(1H-benzotriazol-1-yl) methylpiperidine obtained in Reference Example 5, 4.0 g of 2-bromo-1-(2,4,5-trimethoxyphenyl) propanone, and 2.4 ml of triethylamine were subjected to reaction in 50 ml of methanol in the same manner as in Example 3, and then the reaction mixture was also subjected to the same post-treatment to obtain a crude product. The crude product was purified by chromatography (eluted with $CHCl_3$ : MeOH = 50 : 1) to obtain 1.6 g of 1-[2-oxo-2-(2,4,5-trimethoxyphenyl)-1-methylethyl-4-(1H-benzotriazol-1-yl) methylpiperidine (compound 27).

Example 22

At first, 1.3 g of 4-(1H-benzotriazol-1-yl) methylpiperidine obtained in Reference Example 5, 1.8 g of 2-bromo-1-(3,4-dimethoxyphenyl) propanone, and 1.2 ml of triethylamine were subjected to reaction in 30 ml of methanol in the same manner as in Example 3, and then the reaction mixture was also subjected to the same post-treatment to obtain a crude product. The crude product was purified by chromatography (eluted with $CHCl_3$ : MeOH = 50 : 1) to obtain 0.53 g of 1-[2-oxo-2-(3,4-dimethoxyphenyl)-1-methylethyl-4-(1H-benzotriazol-1-yl) methylpiperidine (compound 30).

Example 23

At first, 0.38 g of the compound obtained in Example 20 was reacted with 0.20 g of sodium borohydride in 15 ml of methanol in the same manner as in Example 2, and then the reaction mixture was also subjected to the same post-treatment to obtain 0.29 g of threo-1-[2-hydroxy-2-(3,4,5-trimethoxyphenyl)-1-methylethyl]-4-[1H-benzotriazol-1-yl) methylpiperidine (compound 34).

Example 24

At first, 1.0 g of the compound obtained in Example 21 was reacted with 0.50 g of sodium borohydride in 50 ml of methanol in the same manner as in Example 8, and then the reaction mixture was also subjected to the same post-treatment. Fractions eluted at first by silica gel column chromatography (eluted with $CHCl_3$ : MeOH = 50 : 1) were collected, and the solvent was removed therefrom by distillation to obtain 0.48 g of threo-1-[2-hydroxy-2-(2,4,5-trimethoxyphenyl)-1-methylethyl]-4-[1H-benzotriazol-1-yl) methylpiperidine (compound 29).

Example 25

Fractions eluted later by the column chromatography of Example 24 were collected, and the solvent was removed therefrom by distillation to obtain 0.33 g of erythro-1-[2-hydroxy-2-(2,4,5-trimethoxyphenyl)-1-methylethyl]-4-[1H-benzotriazol-1-yl) methylpiperidine (compound 28).

Example 26

At first, 0.53 g of the compound obtained in Example 22 was reacted with 0.30 g of sodium borohydride in 30 ml of methanol in the same manner as in Example 8, and then the reaction mixture was also subjected to the same post-treatment. Fractions eluted first by silica gel column chromatography (eluted with $CHCl_3$ : MeOH = 50 : 1) were collected, and the solvent was removed therefrom by distillation to obtain 0.39 g of threo-1-[2-hydroxy-2-(3,4-dimethoxyphenyl)-1-methylethyl]-4-[1H-benzotriazol-1 yl) methylpiperidine (compound 31).

Example 27

Fractions eluted later by the column chromatography of Example 26 were collected, and the solvent was removed therefrom by distillation to obtain 0.11 g of erythro-1-[2-hydroxy-2-(3,4-dimethoxyphenyl)-1-methylethy]-4-[1H-benzotriazol-1-yl) methylpiperidine (compound 32).

Example 28

A solution containing 0.87 g of 4-(2-amino-1H-benzimidazol-1-yl) piperidine dihydrochloride, 0.91 g of 2-bromo-1-(3,4,5-trimethoxyphenyl) propanone and 2.1 ml of triethylamine in 20 ml of methanol was stirred at room temperature for 24 hours, and then the reaction mixture was subjected to the same post-treatment as in Example 3 to obtain 0.61 g of 1-[2-oxo-2-(3,4,5-trimethoxyphenyl-1-methylethyl]-4-(2-amino-1H-benzimidazol-1-yl) piperidine (compound 35).

Example 29

At first, 0.36 g of the compound obtained in Example 28 was reacted with 0.15 g of sodium borohydride in 10 ml of methanol in the same manner as in Example 8, and then the reaction mixture was also subjected to the same post-treatment to obtain 0.13 g of threo-1-[2-hydroxy-2-(3,4,5-trimethoxyphenyl-1-methylethyl]-4-(2-amino-1H-benzimidazol-1-yl) piperidine (compound 36).

Reference Example 1

Synthesis of starting material for Example 1

A suspension containing 70 g of 1-benzyl-4-aminomethylpiperidine [whose synthetic method is disclosed in J. Med. Chem., 9, 441 (1966)], 54.5 g of 2-chloronitrobenzene, 48 g of potassium carbonate and 2.8 g of potassium iodide in 220 ml of dimethylformamide was heated at 150°C for 18 hours. After cooling, the precipitates

were removed therefrom by filtration, and the filtrate was concentrated under reduced pressure. The residue was distributed with ethyl acetate and water. The organic layer was washed with water, dried over magnesium sulfate and concentrated. The residue was dissolved in 300 ml of isopropanol, 40 g of fumaric acid was added thereto, and the mixture was stirred. The resulting crystals were collected therefrom by filtration, washed with isopropanol and ethyl acetate, and dried. The crystals were recrystallized from isopropanol to obtain 115.3 g of 1-benzyl-4-(2-nitrophenylaminomethyl) piperidine fumarate.

Melting point: 179 - 182°C

Elemental analysis as $C_{23}H_{27}N_3O_6$:

Calculated: C = 62.57, H = 6.16, N = 9.52

Found: C = 62.47, H = 6.27, N = 9.52

The compound thus obtained was mixed with an aqueous sodium hydrogen carbonate solution and ethyl acetate by shaking, and the organic layer was separated. The organic layer was washed with water, dried over magnesium sulfate, and concentrated. The free form of the said compound was obtained as the residue.

A mixture of 21.3 g of 1-benzyl-4-(2-nitrophenylaminomethyl) piperidine and 2 g of 10% palladium-carbon in 500 ml of methanol was stirred at room temperature in a hydrogen gas stream for 6 hours. The catalyst was removed therefrom by filtration, and the mother liquor was concentrated. As the residue, 18.6 g of 1-benzyl-4-(2-aminophenylaminomethyl) piperidine was obtained in the form of oily matter.

Infrared absorption spectrum (in $CHCl_3$): 1618, 1504, 1260 $cm^{-1}$

A solution containing 18.5 g of 1-benzyl-4-(2-aminophenylaminomethyl) piperidine and 14 g of 1,1'-carbonyldiimidazole in 200 ml of acetonitrile was refluxed for 12 hours. The reaction mixture was concentrated, and the residue was distributed with chloroform and water. The chloroform layer was washed with an aqueous sodium hydrogen

carbonate solution, and then with water, and dried over magnesium sulfate. Chloroform was removed therefrom by distillation, and the residue was purified by silica gel chromatography (eluent: chloroform : methanol = 10 : 1). The eluate was concentrated, and the residue was recrystallized from isopropanol to obtain 6.76 g of 1-benzyl-4-(1,3-dihydro-2-oxo-2H-benzimidazol-1-yl) methylpiperidine.

Melting point: 169.5 - 170°C

Infrared absorption spectrum (KBr): 1700 (shoulder), 1695 $cm^{-1}$

Elemental analysis as $C_{20}H_{23}N_3O$:

Calculated: C = 74.74, H = 7.21, N = 13.07

Found : C = 74.85, H = 7.41, N = 12.93

A suspension of 5.7 g of 1-benzyl-4-(1,3-dihydro-2-oxo-2H-benzimidazol-1-yl) methylpiperidine, 18 ml of 1 N hydrochloric acid, 0.6 g of 10% palladium-carbon, 50 ml of methanol and 25 ml of water was shaken under a hydrogen pressure of 30 psi at 40°C for 24 hours. The catalyst was removed therefrom by filtration, and the filtrate was concentrated. The residue was recrystallized from isopropanol to obtain 4.4 g of 4-(1,3-dihydro-2-oxo-2H-benzimidazol-1-yl) methylpiperidine hydrochloride.

Melting point: 256 - 258°C

Infrared absorption spectrum (KBr): 1660 $cm^{-1}$

Elemental analysis as $C_{13}H_{17}N_3O \cdot HCl$:

Calculated: C = 58.32, H = 6.78, N = 15.69

Found : C = 58.42, H = 6.93, N = 15.49

Reference Example 2

Synthesis of starting material for Example 5

A solution containing 8.0 g of 4-(2-nitroanilino) piperidine hydrochloride (whose synthetic method is disclosed in Japanese Published Examined Patent Application No. 7267/1972), 8.5 g of 2-bromo-3,4-dimethoxyphenylpropanone and 6.4 g of triethylamine in 200 ml of methanol was stirred at room temperature for 24 hours. The residue was distributed with chloroform and water, and the chloroform

layer was washed with water and dried over magnesium sulfate. The solvent was removed therefrom by distillation, and the residue was recrystallized from ethanol to obtain 8.4 g of 1-[2-(3,4-dimethoxyphenyl)-2-oxo-1-methylethyl]-4-(2-nitroanilino) piperidine.

Melting point: 170 - 174°C

Infrared absorption spectrum (KBr): 1665, 1618, 1570, 1508, 1268 $cm^{-1}$

Elemental analysis as $C_{22}H_{27}N_3O_5$:

Calculated: C = 63.90, H = 6.58, N = 10.16

Found : C = 63.88, H = 6.79, N = 10.15

A suspension of 7.0 g of the product obtained in the above reaction, 3.3 g of acetic acid and 1.0 g of 10% palladium-carbon in 200 ml of methanol was stirred at 35°C for 4 hours while introducing hydrogen gas therein. The catalyst was removed therefrom by filtration, and the filtrate was concentrated under reduced pressure. As the residue, 7.6 g of 1-[2-(3,4-dimethoxyphenyl)-2-oxo-1-methylethyl]-4-(2-aminoanilino) piperidine triacetate was obtained.

Infrared absorption spectrum (KBr): disappearance of the absorption at 1618 and 1570 $cm^{-1}$

The compound was unstable, and thus used as such in the reaction of Example 5.

<u>Reference Example 3</u>

Synthesis of starting material for Example 7

A solution of 6.5 g of 1-benzyl-4-ethoxycarbonyl-3-oxopiperidine and 2.7 g of o-phenylenediamine in 200 ml of m-xylene was subjected to reaction in an Dean-Stark apparatus under the reflux condition for 3 hours. After cooling, the solvent was removed therefrom by distillation, and the residue was recrystallized from m-xylene and ethyl ether, to obtain 5.4 g of 1-benzyl-3-(1,3-dihydro-2-oxo-2H-benzimidazol-1-yl) piperidine-3-en.

Melting point: 155 - 156°C

A mixture of 11.0 g of the compound, 2.2 g of palladium-carbon and 70 ml of 1 N hydrochloric acid in 140 ml of methanol was shaken at 40°C under a hydrogen pressure of 40 psi for 6 days. The catalyst was removed therefrom by filtration, and the filtrate was concentrated. The residue was crystallized from methanol-chloroform to obtain 7.3 g of 3-(1,3-dihydro-2-oxo-2H-benzimidazol-1-yl) piperidine hydrochloride.

Melting point: 270 - 287°C

Infrared absorption spectrum (KBr): 1685, 1480 $cm^{-1}$

Reference Example 4

Synthesis of starting material for Examples 16 and 18

A solution of 33.5 g of 1-benzyl-3-methyl-4-oxopiperidine [whose synthesis method is disclosed in J. Med. Pharm. Chem., 5, 913 (1962)], 127 g of ammonium acetate and 7.3 of sodium cyanoborohydride in 800 ml of methanol was stirred at room temperature for 1.5 hours. Concentrated hydrochloric acid was added thereto to make pH of the reaction mixture 2, and then the reaction mixture was distributed with ethyl acetate and water. Solid sodium hydroxide was added to the water layer to make pH 11, and then the water layer was distributed with chloroform and water. The organic layer was dried over sodium sulfate, and then the solvent was removed therefrom by distillation.

The residue was purified by silica gel chromatography (eluent, chloroform : methanol : aqueous ammonia = 100 : 10 : 1) to obtain 26.0 g of an approximate 1 : 1 mixture of trans-1-benzyl-3-methyl-4-aminopiperidine and cis-1-benzyl-3-methyl-4-aminopiperidine.

Infrared absorption spectrum (KBr): 1560, 1470 $cm^{-1}$

A solution of 26.0 g of the mixture, 22.0 g of o-nitrochlorobenzene, 9.7 g of potassium carbonate and 0.2 g of potassium iodide in 200 ml of dimethylformamide was stirred at 140°C for 3 days. The deposited salt was removed therefrom by filtration, and the filtrate was

concentrated. The residue was distributed with ethyl acetate and water. The organic layer was dried over sodium sulfate, and then the solvent was removed therefrom by distillation. The residue was purified by silica gel chromatography (eluent, chloroform : methanol = 97 : 3) to obtain 18.0 g of an approximate 1 : 1 mixture of trans-1-benzyl-3-methyl-4-(2-nitroanilino) piperidine and cis-1-benzyl-3-methyl-4-(2-nitroanilino) piperidine.

Infrared absorption spectrum (KBr): 1610, 1570, 1500, 1350 $cm^{-1}$

A mixture of 15.8 g of the mixture and 15.0 g of Raney nickel in 2 ℓ of methanol was stirred at room temperature in a hydrogen stream for 6 hours. The catalyst was removed therefrom by filtration, and the fitrate was concentrated.

Then, 31.0 g of 1,1'-carbonyldiimidazole, 9 ml of triethylamine and 700 ml of acetonitrile were added to the residue, and the solution was stirred at 70°C for 1.5 hours. The solvent was removed therefrom by distillation, and the residue was distributed with ethyl acetate and water. The organic layer was dried, and then the solvent was removed by distillation. The residue was subjected to silica gel column chromatography (eluent, ethyl acetate : chloroform = 1 : 4 - 3 : 2), and fractions eluted at first were collected. The solvent was removed therefrom by distillation, and the residue was crystallized from ethyl ether to obtain 5.0 g of cis-1-benzyl-3-methyl-4-(1,3-dihydro-2-oxo-2H-benzimidazol-1-yl) piperidine.

Melting point: 239 - 246°C

Infrared absorption spectrum (KBr): 1690, 1480 $cm^{-1}$

Fractions eluted later by the column chromatography in the reaction were collected, and the solvent was removed therefrom by distillation to obtain 5.6 g of trans-1-benzyl-3-methyl-4-(1,3-dihydro-2-oxo-2H-benzimidazol-1-yl) piperidine.

Infrared absorption spectrum (KBr): 1690, 1480 $cm^{-1}$

A mixture of 4.0 g of trans-1-benzyl-3-methyl-4-(1,3-dihydro-2-oxo-2H-benzimidazol-1-yl) piperidine, 23 ml of 1 N hydrochloric acid, and 2.5 g of 10% palladium-carbon in 100 ml of water and 100 ml of methanol was shaken at room temperature under a hydrogen pressure of 40 psi for 4 days. The catalyst was removed therefrom by filtration, and the filtrate was concentrated to obtain 2.2 g of trans-3-methyl-4-(1,3-dihydro-2-oxo-2H-benzimidazol-1-yl) piperidine hydrochloride.

Infrared absorption spectrum (KBr): 1690, 1480 $cm^{-1}$

A mixture of 4.0 g of the cis-1-benzyl-3-methyl-4-(1,3-dihydro-2-oxo-2H-benzimidazol-1-yl) piperidine, 23 ml of 1 N hydrochloric acid and 2.5 g of 10% palladium-carbon in 80 ml of water and 150 ml of methanol was shaken at room temperature under a hydrogen pressure of 40 psi for 2 days. The catalyst was removed therefrom by filtration, and the filtrate was concentrated. The residue was crystallized from cold water to obtain 2.2 g of cis-methyl-4-(1,3-dihydro-2-oxo-2H-benzimidazol-1-yl) piperidine hydrochloride.

Infrared absorption spectrum (KBr): 1690, 1480 $cm^{-1}$

Reference Example 5

Synthesis of starting material for Examples 20, 21 and 22

A mixture of 2.0 g of 1-benzyl-4-(2-nitrophenylaminomethyl) piperidine and 0.27 g of 10% palladium-carbon in 20 ml of methanol and 20 ml of water was stirred at room temperature under a hydrogen stream for 2 hours. The catalyst was removed therefrom by filtration, and the filtrate was concentrated.

Then, 20 ml of acetic acid and 20 ml of water were added to the residue, and 0.4 g of sodium nitrite was added to the resulting solution at 5°C by portions. The mixture was stirred for 1.5 hours. The solvent was removed therefrom by distillation, and the residue was purified by silica gel column chromatography (eluent, chloroform : methanol = 99 : 1) to obtain 0.58 g of 1-benzyl-

4-(1H-benzotriazol-1-yl) methylpiperidine as crystal.

Melting point: 129 - 130.5°C

Elemental analysis as $C_{19}H_{22}N_4$:

Calculated: C = 74.48, H = 7.24, N = 18.29

Found : C = 74.66, H = 7.25, N = 18.27

A mixture of 15.0 g of the compound and 1.5 g of 10% palladium-carbon in 60 ml of 1 N hydrochloric acid and 100 ml of methanol was shaken at 40°C under a hydrogen pressure of 40 psi for 2 days. The catalyst was removed therefrom by filtration, and the filtrate was concentrated. A concentrated aqueous sodium hydroxide solution was added to the residue to make pH 11, and the mixture was distributed with ethyl acetate and water. The organic layer was dried over sodium sulfate, and the solvent was removed therefrom by distillation to obtain 5.7 g of 4-(1H-benzotriazol-1-yl) methylpiperidine.

Melting point: 139 - 142°C

Infrared absorption spectrum (KBr): 1590, 1450 $cm^{-1}$

Reference Example 6

Synthesis of starting material for Example 28

A solution of 0.55 g of 1-t-butoxycarbonyl-4-(2-aminoanilino) piperidine [whose synthetic method is disclosed in Japanese Published Unexamined Patent Application No. 13780/1976 (published February 3, 1976)] and phenylacetyl isothiocyanate [whose synthesis method is disclosed in Chem. Pharm. Bull., 29, 1832 (1981)] in 10 ml of tetrahydrofuran was stirred at room temperature for 10 minutes. The solvent was removed therefrom by distillation, and the residue was crystallized from ether — n-hexane to obtain 0.38 g of 1-[2-(1-t-butoxycarbonylpiperidin-4-yl) aminophenyl]-2-phenylacetylthiourea.

Melting point: 167°C

Infrared absorption spectrum (KBr): 1680, 1530, 1140 $cm^{-1}$

A suspension of 9.1 g of the compound and 50 ml of concentrated aqueous ammonia in 50 ml of methanol was

subjected to reaction under a reflux condition for one hour. The solvent was removed therefrom by distillation, and the residue was crystallized from water to obtain 6.5 g of 1-[2-(1-t-butoxycarbonylpiperidin-4-yl) aminophenyl] thiourea.

Melting point: 182 - 182.5°C

A solution of 6.0 g of the compound and 2.2 ml of methyl iodide in 100 ml of methanol was stirred at room temperature for 2.5 hours. The solvent was removed therefrom by distillation, and 15 ml of water and 50 ml of methanol were added to the residue.
Then, 2.0 g of Diaion PA412 (OH form) was added thereto, and the mixture was stirred at room temperature for 2 days. The resin was removed therefrom by filtration, and the filtrate was concentrated. The residue was crystallized from water to obtain 5.4 g of 1-t-butoxycarbonyl-4-(2-amino-1H-benzimidazol-1-yl) piperidine.

Melting point: 218 - 220°C

Infrated absorption spectrum (KBr): 1690, 1660, 1420 $cm^{-1}$

Then, 20 ml of a 2.6 N hydrochloric acid-ethyl acetate solution was added to a solution of 4.7 g of the compound in 50 ml of ethyl acetate, and the mixture was stirred at room temperature for 24 hours. The solvent was removed therefrom by distillation, and the residue was crystallized from ethyl acetate to obtain 3.7 g of 4-(2-amino-1H-benzimidazol-1-yl) piperidine dihydrochloride.

Melting point: 284 - 300°C

Infrared absorption spectrum (KBr): 1650, 1470 $cm^{-1}$

## CLAIMS

1. Piperidine derivatives of the formula:

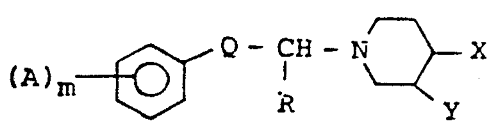

[ I ]

wherein A is a hydroxyl group, a halogen atom, a $C_1$-$C_5$ alkyl group, a $C_1$-$C_5$ alkoxy group, a $C_2$-$C_5$ alkenyloxy group, a $C_1$-$C_5$ alkinyloxy group, a $C_1$-$C_5$ alkylthio group, a carboxyl group, a $C_1$-$C_5$ alkoxycarbonyl group, a nitro group, an amino group, a $C_1$-$C_5$ alkylamino group, a $C_1$-$C_5$alkanoylamino group, a sulfamoyl group, a mono- or di- ($C_1$-$C_5$ alkyl) aminosulfonyl group, a $C_1$-$C_5$ alkylsulfonyl group, a carbamoyl group, a cyano group or a trifluoromethyl group; when m is 2 or more, A may be the same or different groups, or two A groups may combine to form a $C_1$-$C_5$ alkylenedioxy group; m is 0 or an integer of 1-5; Q is a carbonyl group or a hydroxymethylene group; R is a hydrogen atom or an alkyl group having 1-4 carbon atoms; X and Y are defined as follows: i) when Y =H, X represents

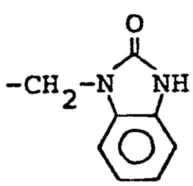 , 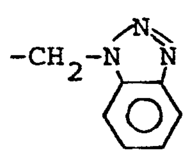 , 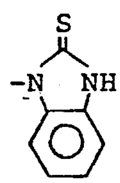 , 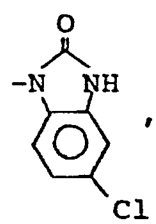 , or 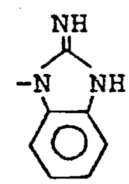

ii) when Y = $CH_3$, X represents 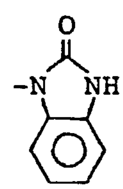 , and

iii) when Y = 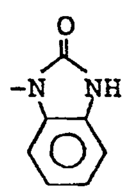 X represents a hydrogen atom

2. Compounds according to claim 1, being compounds of the formula

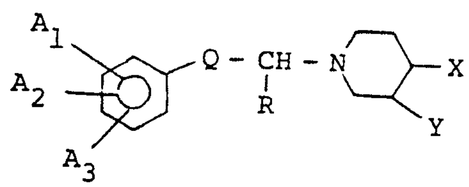

[ I' ]

where $A_1$, $A_2$ and $A_3$ are the same or different groups, and represent hydrogen atoms or $C_1$-$C_5$ alkyloxy groups, and Q, R, X and Y have the same meanings as defined in claim 1.

3. A pharamcologically acceptable acid addition salt of a compound of the formula defined in claim 1 or 2.

4. A salt according to claim 3 being any one of the following: the hydrochloride, hydrobromide, hydroiodide, nitrate, sulfate, phosphate, formate, acetate, benzoate, maleate, fumarate, succinate, tartrate, citrate, oxalate, glyoxylate, aspartate, methanesulfonate, ethanesulfonate, propanesulfonate, methandisulfonate, -ethandisulfonate or benzenesulfonate of a compound of the formula defined in claim 1 or 2.

5. A pharmaceutical composition comprising a pharmaceutically acceptable carrier, and, in admixture therewith, an hypotensive piperidine derivative, characterised in that the hypotensive piperidine derivative is or comprises a compound or salt as claimed in any one of claims 1-4